# EUROPEAN PATENT SPECIFICATION

(11) **EP 4 322 276 B1**
(45) Date of publication and mention of the grant of the patent: **31.12.2025**
(21) Application number: 22893262.0
(22) Date of filing: 11.11.2022
(51) Int. Cl.: H01M 10/0567, H01M 10/052, H01M 10/0569, H01M 10/0525

(54) **NON-AQUEOUS ELECTROLYTE CONTAINING NON-AQUEOUS ELECTROLYTE ADDITIVE, AND LITHIUM SECONDARY BATTERY INCLUDING SAME**
NICHTWÄSSRIGER ELEKTROLYT MIT ADDITIV FÜR NICHTWÄSSRIGEN ELEKTROLYT UND LITHIUMSEKUNDÄRBATTERIE DAMIT
ÉLECTROLYTE NON AQUEUX CONTENANT UN ADDITIF ÉLECTROLYTIQUE NON AQUEUX, ET BATTERIE SECONDAIRE AU LITHIUM LE COMPRENANT

(30) Priority: 12.11.2021 KR 20210155935
(43) Date of publication of application: 14.02.2024
(73) Proprietor: LG Energy Solution, Ltd., Seoul 07335 (KR)
(72) Inventor: YEOM, Chul Eun, Daejeon 34122 (KR); LEE, Chul Haeng, Daejeon 34122 (KR); LEE, Kyung Mi, Daejeon 34122 (KR); LEE, Jung Min, Daejeon 34122 (KR); HAN, Jung Gu, Daejeon 34122 (KR)
(74) Representative: Hoffmann Eitle
(86) International application number: PCT/KR2022/017738
(87) International publication number: WO 2023/085843

(56) References cited:
- CN-A- 104 409 769
- DE-A1- 2 356 900
- JP-A- 2005 347 222
- JP-A- 2005 347 222
- JP-B2- 3 680 454
- KR-A- 20190 123 075
- KR-A- 20210 060 330
- YANG ZAN ET AL: "Electrochemically Induced Thiocyanation of Enaminones: Synthesis of Functionalized Alkenes and Chromones", SYNTHESIS, vol. 52, no. 05, 14 November 2019 (2019-11-14), STUTTGART, DE., pages 711 - 718, XP093065020, ISSN: 0039-7881, DOI: 10.1055/s-0039-1691486
- YANG ZAN, WANG YANHONG, HU LIPING, YU JIANGJING, LI AN, LI LIJUN, YANG TAO, ZHOU CONGSHAN: "Electrochemically Induced Thiocyanation of Enaminones: Synthesis of Functionalized Alkenes and Chromones", SYNTHESIS, GEORG THIEME VERLAG, STUTTGART, DE., vol. 52, no. 05, 1 March 2020 (2020-03-01), STUTTGART, DE. , pages 711 - 718, XP093065020, ISSN: 0039-7881, DOI: 10.1055/s-0039-1691486

## Description

### TECHNICAL FIELD

### Cross-reference to Related Applications

This application claims the benefit of Korean Patent Application No. 10-2021-0155935, filed on November 12, 2021, in the Korean Intellectual Property Office.

### Technical Field

The present invention relates to a non-aqueous electrolyte including an additive for a non-aqueous electrolyte, and a lithium secondary battery including the non-aqueous electrolyte.

### BACKGROUND ART

Recently, as the application area of lithium secondary batteries has rapidly expanded to the power supply of electronic devices such as electricity, electronics, communications, and computers, as well as the power storage supply of large-area devices such as automobiles and power storage devices, the demand for high-capacity, high-output, and high-stability secondary batteries is increasing.

Particularly, in lithium secondary batteries for automotive applications, the properties of high capacity, high power, and long lifespan are becoming important. For the high capacity of a secondary battery, a positive electrode active material with a high content of nickel which is high in energy density but low in stability may be used, or the secondary battery may be driven at a high voltage.

However, when a secondary battery is driven under the above condition, as charge and discharge proceeds, a film formed on surfaces of positive/negative electrodes or the surface structure of an electrode is deteriorated by a side reaction caused by the deterioration of an electrolyte, so that transition metal ions may be eluted from the surface of the positive electrode. As described above, the eluted transition metal ions are electro-deposited on the negative electrode and cause the passivation capability of SEI to degrade, so that there is a problem in that the negative electrode is deteriorated.

The deterioration phenomenon of a secondary battery tends to accelerate when the potential of a positive electrode increases, or when the battery is exposed to high temperatures.

In addition, when a lithium ion battery is continuously used for a long period of time or left to stand at high temperatures, gas is generated and causes a so-called swelling phenomenon in which the thickness of the battery increases, and it is known that the amount of gas generated at this time depends on the state of the SEI.

Therefore, in order to solve the above problem, research and development are being conducted on methods capable of suppressing the elution of metal ions from a positive electrode and forming a stable SEI film on a negative electrode, thereby reducing the swelling phenomenon of a secondary battery, and increasing the stability at high temperatures.

KR 2019-0123075 describes a metal air battery. The metal air battery includes a porous positive electrode, a negative electrode, and an electrolyte solution located between the positive electrode and the negative electrode, and the electrolyte solution contains a solvent, a metal salt, a liquid catalyst, and a singlet oxygen scavenger.

JP 2005347222 relates to an electrolytic solution and a battery using the electrolytic solution, and more particularly to an electrolytic solution and a battery that can function when propylene carbonate is contained.

### DISCLOSURE OF THE INVENTION

### TECHNICAL PROBLEM

An aspect of the present invention provides an additive for a non-aqueous electrolyte capable of suppressing the degradation of a positive electrode, reducing side reactions between a positive electrode and an electrolyte, and forming a stable SEI film on a negative electrode.

Another aspect of the present invention provides a non-aqueous electrolyte having improved stability at high temperatures by including the additive for a non-aqueous electrolyte.

Another aspect of the present invention provides a lithium secondary battery with improved overall performance by including the non-aqueous electrolyte, thereby having improved high-temperature cycle properties and high-temperature storage properties.

### TECHNICAL SOLUTION

According to an aspect of the present invention, there is provided a non-aqueous electrolyte including an additive for a non-aqueous electrolyte represented by Formula 1 below.

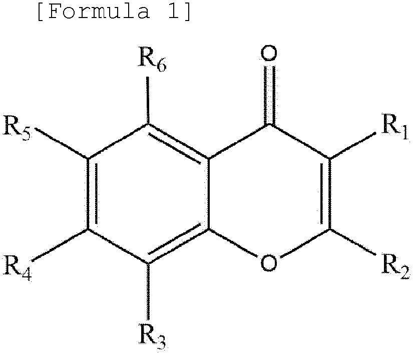

In Formula 1 above, R₁ and R₂ may each independently be any one selected from the group consisting of H; F; a nitrile group; a thiocyanate group; an isothiocyanate group; an isocyanate group; an alkoxycarbonyl group having 1 to 10 carbon atoms; an alkylcarbonyl group having 1 to 10 carbon atoms; a sulfonyl group substituted with an alkyl group having 1 to 10 carbon atoms, an alkenyl group having 2 to 10 carbon atoms, or an alkynyl group having 2 to 10 carbon atoms; an oxysulfonyl group substituted with an alkyl group having 1 to 10 carbon atoms, an alkenyl group having 2 to 10 carbon atoms, or an alkynyl group having 2 to 10 carbon atoms; CF₃; OCF₃;COOCF₃; and SO₂CF₃, wherein R₁ and R₂ are not H at the same time, and R₃ to R₆ may each independently be any one selected from the group consisting of H, an alkyl group having 1 to 10 carbon atoms, an alkenyl group having 2 to 10 carbon atoms, an alkynyl group having 2 to 10 carbon atoms, an alkoxy group having 1 to 10 carbon atoms, a cycloalkyl group having 3 to 12 carbon atoms, a cycloalkenyl group having 3 to 12 carbon atoms, and an aryl group having 6 to 20 carbon atoms,
wherein the alkoxycarbonyl is a -COOR, in which R is an alkyl group having 1 to 10 carbon atoms, and, wherein the non-aqueous electrolyte further comprises a lithium salt.

According to another aspect of the present invention, there is provided a lithium secondary battery including the non-aqueous electrolyte of the present invention.

### ADVANTAGEOUS EFFECTS

A compound represented by Formula 1 above provided as an additive for a non-aqueous electrolyte according to the present invention has a basic structure of chromone. The compound having a chromone structure is capable of suppressing the decomposition of an electrolyte by preferentially reacting with a superoxide generated due to the structural collapse of a positive electrode active material at a high voltage.

Particularly, chromone has an electron density biased toward oxygen, which has high electron affinity, and thus, is characterized by having high reactivity at positions 2 and 3 which have relatively low electron density. The compound represented by Formula 1 above of the present invention has an electron withdrawing group substituted at positions 2 and 3 of chromone, so that reactivity with a superoxide may be further increased, and the superoxide may be more effectively removed.

In addition, the compound represented by Formula 1 of the present invention may form a film on an electrode to protect positive/negative electrodes, and may form an organic film to lower battery resistance. That is, the compound represented by Formula 1 of the present invention may form a stable solid electrolyte interphase (SEI) film on the surface of a negative electrode while minimizing an increase in the resistance of a lithium secondary battery. Therefore, since it is possible to prevent the negative electrode from deteriorating by suppressing the degradation in passivation capability of the SEI at high temperatures, the lifespan properties of the battery may be improved.

That is, when a non-aqueous electrolyte of the present invention including the compound of Formula 1 above is used, it is possible to form an electrode-electrolyte interface which is stable at high temperatures and low in resistance, so that high-temperature cycle properties and high-temperature storage properties are improved to implement a lithium secondary battery with improved overall performance.

### MODE FOR CARRYING OUT THE INVENTION

It will be further understood that the words or terms should be interpreted as having meanings that are consistent with their meanings in the context of the relevant art and the technical idea of the invention, based on the principle that an inventor may properly define the meaning of the words or terms to best explain the invention.

In the present specification, it should be understood that the terms "include," "comprise," or "have" are intended to specify the presence of stated features, numbers, steps, elements, or combinations thereof, but do not preclude the presence or addition of one or more other features, numbers, steps, elements, or combinations thereof.

In addition, in the present specification, it will be understood that in the description of "carbon atoms a to b" herein, "a" and "b" refer to the number of carbon atoms included in a specific functional group. That is, the functional group may include "a" to "b" number of carbon atoms. For example, an "alkylene group having 1 to 5 carbon atoms" refers to an alkylene group including carbon atoms with a carbon number of 1 to 5, that is, -CH₂-, -CH₂CH₂-, -CH₂CH₂CH₂-, -CH₂(CH₃)CH-, -CH(CH₃)CH₂-, -CH(CH₃)CH_{2C}H₂-, and the like.

In addition, in the present disclosure, the term "alkylene group" refers to a branched or unbranched divalent hydrocarbon group.

In addition, in the present specification, an alkyl group or an alkylene group may all be substituted or unsubstituted. Unless otherwise defined, the term "substituted" means that at least one hydrogen bonded to carbon is substituted with an element other than hydrogen, and for example, it means being substituted with an alkyl group having 1 to 20 carbon atoms, an alkenyl group having 2 to 20 carbon atoms, an alkynyl group having 2 to 20 carbon atoms, an alkoxy group having 1 to 20 carbon atoms, a cycloalkyl group having 3 to 12 carbon atoms, a cycloalkenyl group having 3 to 12 carbon atoms, a heterocycloalkyl group having 3 to 12 carbon atoms, a heterocycloalkenyl group having 3 to 12 carbon atoms, an aryloxy group having 6 to 12 carbon atoms, a halogen atom, a fluoroalkyl group having 1 to 20 carbon atoms, a nitro group, an aryl group having 6 to 20 carbon atoms, a heteroaryl group having 2 to 20 carbon atoms, a haloaryl group having 6 to 20 carbon atoms, or the like.

Hereinafter, the present invention will be described in more detail.

### Non-aqueous electrolyte

A non-aqueous electrolyte according to an embodiment of the present invention includes a compound represented by Formula 1 below as an additive and a lithium salt. A secondary battery including the non-aqueous electrolyte of the present invention may have high lithium ion conductivity by having the form of zwitterions, and may have excellent high-temperature cycle properties and high-temperature storage properties since deterioration caused by an interfacial reaction at high temperatures are suppressed.

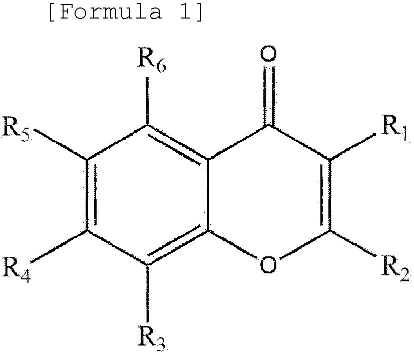

The compound of Formula 1 has a basic structure of chromone. The compound having a chromone structure is capable of suppressing the decomposition of an electrolyte by preferentially reacting with a superoxide generated due to the structural collapse of a positive electrode active material at a high voltage.

Particularly, chromone has an electron density biased toward oxygen, which has high electron affinity, and thus, is characterized by having high reactivity at positions 2 and 3 which have relatively low electron density. The compound represented by Formula 1 above of the present invention has an electron withdrawing group substituted at positions 2 and 3 of chromone, so that reactivity with a superoxide may be further increased, and the superoxide may be more effectively removed.

In addition, the compound represented by Formula 1 of the present invention may form a film on an electrode to protect positive/negative electrodes, and may form an organic film to lower battery resistance. That is, the compound represented by Formula 1 of the present invention may form a stable solid electrolyte interphase (SEI) film on the surface of a negative electrode while minimizing an increase in the resistance of a lithium secondary battery. Therefore, since it is possible to prevent the negative electrode from deteriorating by suppressing the degradation in passivation capability of the SEI at high temperatures, the lifespan properties of the battery may be improved.

In Formula 1 above, R₁ and R₂ may be an electron withdrawing group. In Formula 1 above, R₁ and R₂ may each independently be any one selected from the group consisting of H; F; a nitrile group; a thiocyanate group; an isothiocyanate group; an isocyanate group; an alkoxycarbonyl group having 1 to 10 carbon atoms; an alkylcarbonyl group having 1 to 10 carbon atoms; a sulfonyl group substituted with an alkyl group having 1 to 10 carbon atoms, an alkenyl group having 2 to 10 carbon atoms, or an alkynyl group having 2 to 10 carbon atoms; an oxysulfonyl group substituted with an alkyl group having 1 to 10 carbon atoms, an alkenyl group having 2 to 10 carbon atoms, or an alkynyl group having 2 to 10 carbon atoms; CF₃; OCF₃; COOCF₃; and SO₂CF₃. Here, a case in which R₁ and R₂ are H at the same time is excluded. Specifically, the alkoxycarbonyl group having 1 to 10 carbon atoms refers to -COOR (wherein R is an alkyl group having 1 to 10 carbon atoms). The alkylcarbonyl group having 1 to 10 carbon atoms refers to - COR' (wherein R' is an alkyl group having 1 to 10 carbon atoms) . The sulfonyl group substituted with an alkyl group having 1 to 10 carbon atoms, an alkenyl group having 2 to 10 carbon atoms, or an alkynyl group having 2 to 10 carbon atoms refers to - SO₂R" (wherein R" is an alkyl group having 1 to 10 carbon atoms, an alkenyl group having 2 to 10 carbon atoms, or an alkynyl group having 2 to 10 carbon atoms). The oxysulfonyl group substituted with an alkyl group having 1 to 10 carbon atoms, an alkenyl group having 2 to 10 carbon atoms, or an alkynyl group having 2 to 10 carbon atoms refers to - OSO₂R‴ (wherein R‴ is an alkyl group having 1 to 10 carbon atoms, an alkenyl group having 2 to 10 carbon atoms, or an alkynyl group having 2 to 10 carbon atoms).

In Formula 1 above, R₃ to R₆ may each independently be any one selected from the group consisting of H, an alkyl group having 1 to 10 carbon atoms, an alkenyl group having 2 to 10 carbon atoms, an alkynyl group having 2 to 10 carbon atoms, an alkoxy group having 1 to 10 carbon atoms, a cycloalkyl group having 3 to 12 carbon atoms, a cycloalkenyl group having 3 to 12 carbon atoms, and an aryl group having 6 to 20 carbon atoms. In addition, in Formula 1 above, R₃ to R₆ may each independently be any one selected from the group consisting of H and an alkyl group having 1 to 10 carbon atoms.

Preferably, the compound of Formula 1 above may be a compound represented by Formula 1-1 below.

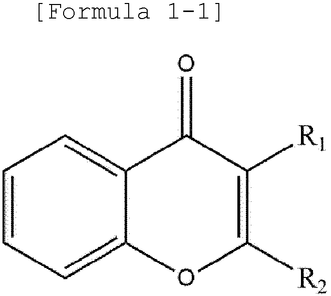

In Formula 1-1 above, R₁ and R₂ may each independently be any one selected from the group consisting of H, F, an alkoxycarbonyl group having 1 to 10 carbon atoms, and a nitrile group. Here, a case in which R₁ and R₂ are H at the same time is excluded.

An additive for a non-aqueous electrolyte according to the present invention may be any one selected from the group consisting of Formulas 1-2a to 1-2h.

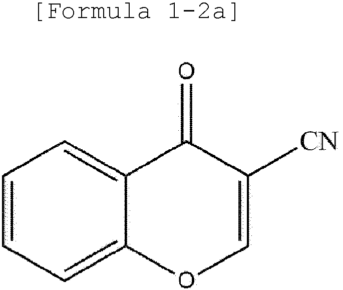

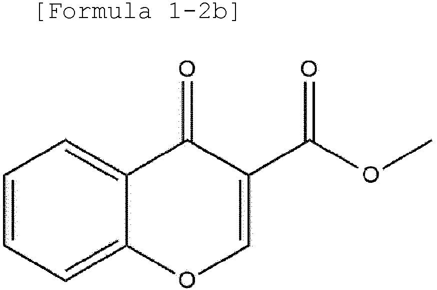

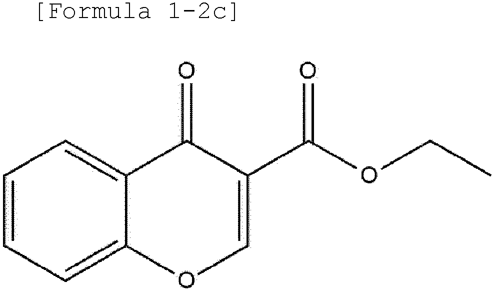

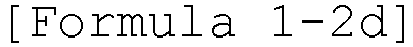

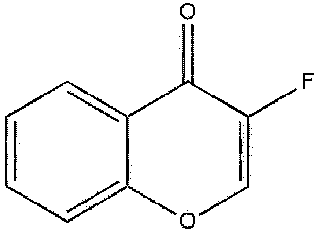

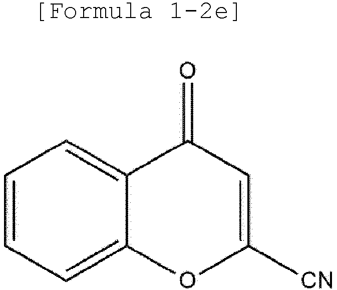

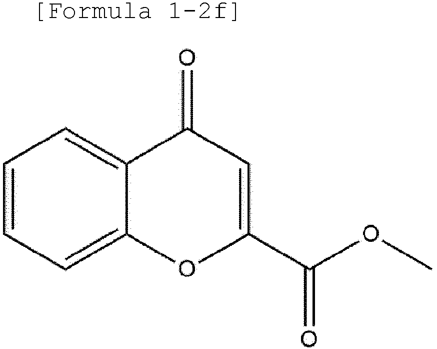

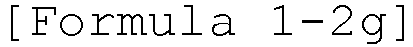

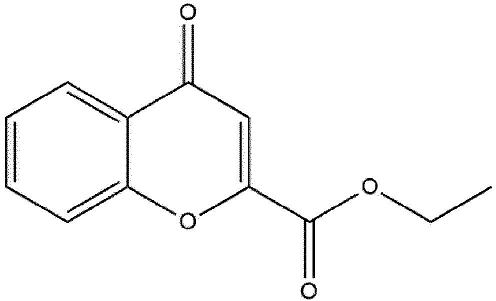

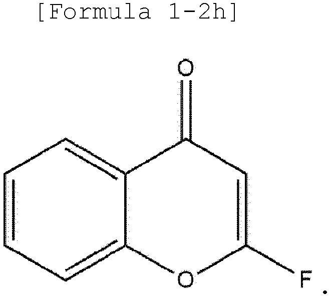

The additive for a non-aqueous electrolyte according to the present invention may be included in an amount of 0.01 parts by weight to 10 parts by weight, preferably 0.1 parts by weight to 5 parts by weight, and more preferably 0.5 parts by weight to 1 parts by weight, based on 100 parts by weight of the non-aqueous electrolyte. When the content of the compound represented by Formula 1 above satisfies the above range, there is a sufficient effect of forming a film on a positive electrode, thereby achieving an effect of suppressing the elution of a transition metal from a positive electrode active material, and the viscosity of the electrolyte is maintained at a suitable level, thereby achieving an effect in that rate properties or lifespan properties are excellent during high-temperature storage.

The non-aqueous electrolyte according to the present invention includes a lithium salt, and may further include an organic solvent, or other electrolyte additives.

The lithium salt is used as an electrolyte salt in a lithium secondary battery, and is used as a medium for transferring ions. Typically, the lithium salt may include Li⁺ as cations, and may include, as anions, at least one selected from the group consisting of F⁻, Cl⁻, Br⁻, I⁻, NO₃⁻, N(CN)₂⁻, BF₄⁻, ClO₄⁻, B₁₀Cl₁₀⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, (CF₃CF₂SO₂)₂N⁻, (CF₃SO₂)₂N⁻, (FSO₂)₂N⁻, BF₂C₂O₄⁻, BC₄O₈⁻, PF₄C₂O₄⁻, PF₂C₄O₈⁻, (CF₃)₂PF₄⁻, (CF₃)₃PF₃⁻, (CF₃)₄PF₂⁻, (CF₃)₅PF⁻, (CF₃)₆P⁻, C₄F₉SO₃⁻, CF₃CF₂SO₃⁻, CF₃CF₂(CF₃)₂CO⁻, (CF₃SO₂)₂CH⁻, CF₃(CF₂)₇SO₃⁻, and SCN⁻.

Specifically, the lithium salt may include a single material selected from the group consisting of LiCl, LiBr, LiI, LiBF₄, LiClO₄, LiB₁₀Cl₁₀, LiAlCl₄, LiAlO₂, LiPF₆, LiCF₃SO₃, LiCH₃CO₂, LiCF₃CO₂, LiAsF₆, LiSbF₆, LiCH₃SO₃, LiN(SO₂F)₂ (lithium bis (fluorosulfonyl) imide (LiFSI)), LiN (SO₂CF₂CF₃)₂ (lithium bis(perfluoroethanesulfonyl)imide (LiBETI)), and LiN (SO₂CF₃)₂ (lithium bis (trifluoromethanesulfonyl) imide (LiTFSI)), or a mixture of two or more thereof. In addition to the above, any lithium salt commonly used in an electrolyte of a lithium secondary battery may be used without limitation.

Although it may be appropriately changed within a typical range in which a lithium salt may be used, in order to obtain an optimum effect of forming an anti-corrosive film on the surface of an electrode, the lithium salt may be included in the electrolyte at a concentration of 0.5 M to 2.0 M, preferably 0.5 M to 1.8 M, more preferably 0.7 M to 1.6 M. When the concentration of the lithium salt satisfies the above range, there is a sufficient effect of improving cycle properties during high-temperature storage of a lithium secondary battery, and the viscosity of the non-aqueous electrolyte is suitable, so that the wettability of the electrolyte may be improved.

The non-aqueous organic solvent may include at least one organic solvent selected from the group consisting of a cyclic carbonate-based organic solvent, a linear carbonate-based organic solvent, a linear ester-based organic solvent, and a cyclic ester-based organic solvent.

Specifically, the organic solvent may include a cyclic carbonate-based organic solvent, a linear carbonate-based organic solvent, or a mixed organic solvent thereof.

The cyclic carbonate-based organic solvent is an organic solvent having high viscosity and a high dielectric constant, and thus, is an organic solvent capable of dissociating a lithium salt well in an electrolyte, and specific examples thereof may include at least one organic solvent selected from the group consisting of ethylene carbonate (EC), propylene carbonate (PC), 1,2-butylene carbonate, 2,3-butylene carbonate, 1,2-pentylene carbonate, 2,3-pentylene carbonate, and vinylene carbonate, and among them, may include ethylene carbonate.

In addition, the linear carbonate-based organic solvent is an organic solvent having low viscosity and a low dielectric constant, and representative examples thereof may include at least one organic solvent selected from the group consisting of dimethyl carbonate (DMC), diethyl carbonate (DEC), dipropyl carbonate, ethylmethyl carbonate (EMC), methylpropyl carbonate, and ethylpropyl carbonate, and specifically, may include ethylmethyl carbonate (EMC).

In addition, in order to prepare an electrolyte having a high ion conductivity, the organic solvent may further include at least one carbonate-based organic solvent selected from the group consisting of the cyclic carbonate-based organic solvent and the linear carbonate-based organic solvent and at least one ester-based organic solvent selected from the group consisting of the linear ester-based organic solvent and the cyclic ester-based organic solvent.

Specific examples of the linear ester-based organic solvent may include at least one organic solvent selected from the group consisting of methyl acetate, ethyl acetate, propyl acetate, methyl propionate, ethyl propionate, propyl propionate, and butyl propionate.

In addition, the cyclic ester-based organic solvent may be at least one organic solvent selected from the group consisting of γ-butyrolactone, γ-valerolactone, γ-caprolactone, σ-valerolactone, and ε-caprolactone.

Meanwhile, if necessary, the organic solvent may additionally use any organic solvent commonly used in a non-aqueous electrolyte without limitation. For example, the organic solvent may further include at least one organic solvent among an ether-based organic solvent, a glyme-based solvent, and a nitrile-based organic solvent.

As the ether-based solvent, any one selected from the group consisting of dimethyl ether, diethyl ether, dipropyl ether, methyl ethyl ether, methyl propyl ether, ethyl propyl ether, 1,3-dioxolane (DOL), and 2,2-bis (trifluoromethyl)-1,3-dioxolane (TFDOL), or a mixture of two or more thereof may be used, but the present invention is not limited thereto.

The glyme-based solvent is a solvent having a higher dielectric constant and lower surface tension than those of a linear carbonate-based organic solvent, and having less reactivity with a metal, and may include at least one selected from the group consisting of dimethoxyethane (glyme, DME), diethoxyethane, diglyme, tri-glyme, and tetra-glyme (TEGDME), but is not limited thereto.

The nitrile-based solvent may be one or more selected from the group consisting of acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, 4-fluorophenylacetonitrile, but is not limited thereto.

In addition, the non-aqueous electrolyte of the present invention may further include, if necessary, an electrolyte additive known in the art in the non-aqueous electrolyte to prevent the non-aqueous electrolyte from being decomposed in a high-output environment and causing a negative electrode to collapse, or to further improve low-temperature high-rate discharge properties, high-temperature stability, overcharge prevention, the effect of suppressing battery expansion at high temperatures, and the like.

Representative examples of the electrolyte additive may include at least one additive for forming an SEI film selected from the group consisting of a cyclic carbonate-based compound, a halogen-substituted carbonate-based compound, a sultone-based compound, a sulfate-based compound, a phosphate-based compound, a borate-based compound, a nitrile-based compound, a benzene-based compound, an amine-based compound, a silane-based compound, and a lithium salt-based compound.

The cyclic carbonate-based compound may be vinylene carbonate (VC) or vinylethylene carbonate.

The halogen-substituted carbonate-based compound may be fluoroethylene carbonate (FEC).

The sultone-based compound may be at least one compound selected from the group consisting of 1,3-propane sultone (PS), 1,4-butane sultone, ethene sulfone, 1,3-propene sultone (PRS), 1,4-butene sultone, and 1-methyl-1,3-propene sultone.

The sulfate-based compound may be ethylene sulfate (ESA), trimethylene sulfate (TMS) or methyl trimethylene sulfate (MTMS).

The phosphate-based compound may be one or more compound selected from lithium difluoro(bisoxalato)phosphate, lithium difluorophosphate, tetramethyl trimethyl silyl phosphate, trimethyl silyl phosphite, tris(2,2,2-trifluoroethyl)phosphate, and tris(trifluoroethyl)phosphite.

The borate-based compound may be tetraphenylborate, lithium oxalyldifluoroborate (LiODFB), and lithium bisoxalatoborate (LiB(C₂O₄)₂, LiBOB).

The nitrile-based compound may be at least one compound selected from the group consisting of succinonitrile, adiponitrile, acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile.

The benzene-based compound may be fluorobenzene, the amine-based compound may be triethanolamine, ethylene diamine, or the like, and the silane-based compound may be tetravinylsilane.

The lithium salt-based compound is a compound different from the lithium salt included in the non-aqueous electrolyte, and may be lithium difluorophosphate (LiDFP), LiPO₂F₂, LiBF₄, or the like.

Among the electrolyte additives, when a combination of vinylene carbonate (VC), 1,3-propane sultone (PS), ethylene sulfate (Esa), and lithium difluorophosphate (LiDFP) is included, it is possible to form a more robust SEI film on the surface of a negative electrode during an initial activation process of a secondary battery, and to suppress the generation of a gas which may be generated due to the decomposition of an electrolyte at high temperatures, thereby improving high-temperature stability of the secondary battery.

Meanwhile, two or more of the electrolyte additives may be mixed and used, and may be 0.01 to 30 wt%, specifically 0.1 to 25 wt%, preferably 1 to 20 wt%, based on the total weight of the non-aqueous electrolyte. When the content of the electrolyte additives satisfies the above range, there is a more excellent effect of improving ion conductivity and cycle properties.

### Lithium secondary battery

The present invention also provides a lithium secondary battery including the non-aqueous electrolyte.

Specifically, the lithium secondary battery includes a positive electrode including a positive electrode active material, a negative electrode including a negative electrode active material, a separator interposed between the positive electrode and the negative electrode, and the above-described non-aqueous electrolyte.

At this time, the lithium secondary battery of the present invention may be manufactured by a common method known in the art. For example, the lithium secondary battery of the present invention may be manufactured by forming an electrode assembly in which a positive electrode, a negative electrode, and a separator between the positive electrode and the negative electrode are sequentially stacked, followed by inserting the electrode assembly in a battery case, and then injecting the non-aqueous electrolyte according to the present invention thereto.

### (1) Positive electrode

The positive electrode may be manufactured by coating a positive electrode mixture slurry including a positive electrode active material, a binder, a conductive material, a solvent, and the like on a positive electrode current collector.

The positive electrode current collector is not particularly limited as long as it has conductivity without causing a chemical change in the battery. For example, stainless steel, aluminum, nickel, titanium, fired carbon, or aluminum or stainless steel that is surface-treated with one of carbon, nickel, titanium, silver, and the like may be used.

The positive electrode active material is a compound capable of reversible intercalation and de-intercalation of lithium, and specifically, may include a lithium metal oxide including one or more metals, such as cobalt, manganese, nickel, and aluminum, and lithium. More specifically, the lithium metal oxide may be a lithium-manganese-based oxide (e.g., LiMnO₂, LiMn₂O₄, etc.), a lithium-cobalt-based oxide (e.g., LiCoO₂, etc.), a lithium-nickel-based oxide (e.g., LiNiO₂, etc.), a lithium-nickel-manganese-based oxide (e.g., LiNi_{1-Y}Mn_{Y}O₂ (wherein 0<Y<1) , LiMn_{2-Z}Ni_{Z}O₄ (wherein 0 < Z < 2), etc.), a lithium-nickel-cobalt-based oxide (e_{.}g_{.}, LiNi_{1-Y1}Co_{Y1}O₂ (wherein 0<Y1<1), etc.), a lithium-manganese-cobalt-based oxide (e.g., LiCo_{1-Y2}Mn_{Y2}O₂ (wherein 0<Y2<1), LiMn_{2-Z1}Co_{Z1}O₄ (wherein 0<Z1<2) , etc.), a lithium-nickel-manganese-cobalt-based oxide (e.g., Li(NiₚCo_{q}Mnᵣ)O₂ (wherein 0<p<1, 0<q<1, 0<r<1, and p+q+r=1) or Li(Niₚ₁Co_{q1}Mnᵣ₁)O₄ (wherein 0<p1<2, 0<q1<2, 0<r1<2, and p1+q1+r1=2), etc.), or a lithium-nickel-cobalt-transition metal (M) oxide (e.g., Li (Niₚ₂Co_{q2}Mnᵣ₂Mₛ₂)O₂ (wherein M is selected from the group consisting of Al, Fe, V, Cr, Ti, Ta, Mg, and Mo, and p2, q2, r2, and s2 are each an atomic fraction of stand-alone elements, wherein 0<p2<1, 0<q2<1, 0<r2<1, 0<s2<1, and p2+q2+r2+s2=1), etc.) and the like, and any one thereof or a compound of two or more thereof may be included.

Among these, due to the fact that the capacity properties and stability of a battery may be increased, the lithium metal oxide may be LiCoO₂, LiMnO₂, LiNiO₂, a lithium nickel-manganese-cobalt oxide (e.g., Li(Ni_{1/3}Mn_{1/3}Co_{1/3})O₂, Li(Ni_{0.6}Mn_{0.2}Co_{0.2})O₂, Li(Ni_{0.5}Mn_{0.3}Co_{0.2})O₂, Li(Ni_{0.7}Mn_{0.15}Co_{0.15})O₂, Li(Ni_{0.8}Mn_{0.1}Co_{0.1})O₂, etc.), a lithium nickel cobalt aluminum oxide (e.g., Li(Ni_{0.8}Co_{0.15}Al_{0.05})O₂, etc.), or the like, and any one thereof or a mixture of two or more thereof may be used.

Among the above, a positive electrode active material having a nickel content of 80 atm% or greater may be used in that capacity properties of a battery may be most increased. For example, the lithium transition metal oxide may include one represented by [Formula 2] below.

[Formula 2] LiₓNiₐCoₑM¹_{c}M²_{d}O₂

In Formula 2 above, the M¹ is one or more selected from Mn and Al, and preferably, may be Mn or a combination of Mn and Al.

The M² is one or more selected from the group consisting of Zr, B, W, Mg, Ce, Hf, Ta, La, Ti, Sr, Ba, F, P, and S.

The x represents an atomic fraction of lithium in a lithium transition metal oxide, wherein the x may satisfy 0.90≤x≤1.1, preferably 0.95≤x≤1.08, more preferably 1.0≤x≤1.08.

The a represents an atomic fraction of nickel among metal elements excluding lithium in a lithium transition metal oxide, wherein the a may satisfy 0.80≤a<1.0, preferably 0.80≤a≤0.95, more preferably 0.80≤a≤0.90. When the nickel content satisfies the above range, it is possible to implement high-capacity properties.

The b represents an atomic fraction of cobalt among metal elements excluding lithium in a lithium transition metal oxide, wherein the b may satisfy 0<b<0.2, 0<b≤0.15, or 0.01≤b≤0.10.

The c represents an atomic fraction of M¹ among metal elements excluding lithium in a lithium transition metal oxide, wherein the c may satisfy 0<c<0.2, 0<c≤0.15, or 0.01≤c≤0.10.

The d represents an atomic fraction of M² among metal elements excluding lithium in a lithium transition metal oxide, wherein the d may satisfy 0≤d≤0.1, or 0≤d≤0.05.

The positive electrode active material may be included in an amount of 60 wt% to 99 wt%, preferably 70 wt% to 99 wt%, more preferably 80 wt% to 98 wt%, based on the total weight of solids excluding the solvent in the positive electrode mixture slurry.

The binder is a component for assisting in coupling between an active material, a conductive material, etc., and coupling to a current collector.

Examples of the binder may include polyvinylidene fluoride, polyvinyl alcohol, carboxymethyl cellulose (CMC), starch, hydroxypropyl cellulose, regenerated cellulose, polyvinylpyrrolidone, polytetrafluoroethylene, polyethylene (PE), polypropylene, an ethylene-propylene-diene monomer, a sulfonated ethylene-propylene-diene monomer, styrene-butadiene rubber, fluorine rubber, various copolymers thereof, and the like.

Typically, the binder may be included in an amount of 1 to 20 wt%, preferably 1 to 15 wt%, more preferably 1 wt% to 10 wt%, based on the total weight of solids excluding the solvent in the positive electrode mixture slurry.

The conductive material is a component for further improving the conductivity of the positive electrode active material, and may be added in an amount of 1 to 20 wt% based on the total weight of solids in the positive electrode mixture slurry. The conductive material is not particularly limited as long as it has conductivity without causing a chemical change in the battery, and for example, carbon powder such as carbon black, acetylene black, Ketjen black, channel black, furnace black, lamp black, or thermal black; graphite powder of natural graphite, artificial graphite, graphite, or the like, which has a very developed crystal structure; conductive fiber such as carbon fiber or metal fiber; conductive powder such as fluorocarbon powder, aluminum powder, and nickel powder; a conductive whisker such as zinc oxide and potassium titanate; a conductive metal oxide such as titanium oxide; a conductive material such as a polyphenylene derivative, and the like may be used.

Typically, the conductive material may be included in an amount of 1 to 20 wt%, preferably 1 to 15 wt%, more preferably 1 wt% to 10 wt%, based on the total weight of solids excluding the solvent in the positive electrode mixture slurry.

The solvent may include an organic solvent such as N-methyl-2-pyrrolidone (NMP), and may be used in an amount such that a preferred viscosity is achieved when the positive electrode active material, and selectively a binder, a conductive material, and the like are included. For example, the solvent may be included in an amount such that the concentration of a solid including the positive electrode active material, and selectively the binder and the conductive material is 50 wt% to 95 wt%, preferably 70 wt% to 95 wt%, more preferably 70 wt% to 90 wt%.

### (2) Negative electrode

The negative electrode may be manufactured by, for example, coating a negative electrode mixture slurry containing a negative electrode active material, a binder, a conductive material, a solvent, and the like on a negative electrode current collector, or a graphite electrode made of carbon (C) or a metal itself may be used as the negative electrode.

For example, when the negative electrode is manufactured by coating a negative electrode mixture slurry on a negative electrode current collector, the negative electrode current collector typically has a thickness of 3 to 500 µm. The negative electrode current collector is not particularly limited as long as it has high conductivity without causing a chemical change in the battery, and for example, copper, stainless steel, aluminum, nickel, titanium, fired carbon, copper or stainless steel that is surface-treated with one of carbon, nickel, titanium, silver, and the like, an aluminumcadmium alloy, and the like may be used. Also, as in the case of the positive electrode current collector, microscopic irregularities may be formed on the surface of the negative electrode current collector to improve the biding force of a negative electrode active material, and the negative electrode current collector may be used in various forms of such as a film, a sheet, a foil, a net, a porous body, a foam body, and a non-woven fabric body.

In addition, the negative electrode active material may include at least one selected from the group consisting of a lithium metal, a carbon material capable of reversible intercalation/de-intercalation of lithium ions, a metal or an alloy of the metal and lithium, a metal composite oxide, a material capable of doping and undoping lithium, and a transition metal oxide.

As the carbon material capable of reversible intercalation/de-intercalation of lithium ions, a carbon-based negative electrode active material commonly used in a lithium ion secondary battery may be used without particular limitation, and representative examples thereof may include a crystalline carbon, an amorphous carbon, or a combination thereof. Examples of the crystalline carbon may include graphite such as an irregular, planar, flaky, spherical, or fibrous natural graphite or artificial graphite, and examples of the amorphous carbon may include soft carbon (low-temperature fired carbon) or hard carbon, mezophase pitch carbides, fired cokes, and the like.

As the metal or the alloy of the metal and lithium, a metal selected from the group consisting of Cu, Ni, Na, K, Rb, Cs, Fr, Be, Mg, Ca, Sr, Si, Sb, Pb, In, Zn, Ba, Ra, Ge, Al, and Sn, or an alloy of the metal and lithium may be used.

As the metal composite oxide, one selected from the group consisting of PbO, PbO₂, Pb₂O₃, Pb₃O₄, Sb₂O₃, Sb₂O₄, Sb₂O₅, GeO, GeO₂, Bi₂O₃, Bi₂O₄, Bi₂O₅, LiₓFe₂O₃(0≤x≤1), LiₓWO₂(0≤x≤1), and SnₓMe₁₋ₓMe'_{y}O_{z} (Me: Mn, Fe, Pb, Ge; Me': Al, B, P, Si, elements in Group 1, Group 2, and Group 3 of the periodic table, halogen; 0<x≤1; 1≤y≤3; 1≤z≤8) may be used.

The material capable of doping and undoping lithium may be Si, SiOₓ(O<x≤2), an Si-Y alloy (wherein Y is an element selected from the group consisting of an alkali metal, an alkaline earth metal, a Group 13 element, a Group 14 element, a transition metal, a rare earth element, and a combination thereof, but not Si), Sn, SnO₂, Sn-Y (wherein Y is an element selected from the group consisting of an alkali metal, an alkaline earth metal, a Group 13 element, a Group 14 element, a transition metal, a rare earth element, and a combination thereof, but not Sn), and the like, or at least one thereof may be mixed with SiO₂ and used. The element Y may be selected from the group consisting of Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Ge, P, As, Sb, Bi, S, Se, Te, Po, and a combination thereof.

The transition metal oxide may be a lithium-containing titanium composite oxide (LTO), a vanadium oxide, a lithium vanadium oxide, and the like.

The negative electrode active material may be included in an amount of 60 to 99 wt%, preferably 70 to 99 wt%, more preferably 80 to 98 wt%, based on the total weight of solids in the negative electrode mixture slurry.

The binder is a component for assisting in coupling between a conductive material, an active material, and a current collector. Examples of the binder may include polyvinylidene fluoride (PVDF), polyvinyl alcohol, carboxymethyl cellulose (CMC), starch, hydroxypropyl cellulose, regenerated cellulose, polyvinylpyrrolidone, polytetrafluoroethylene, polyethylene, polypropylene, an ethylene-propylene-diene monomer, a sulfonated ethylene-propylene-diene monomer, styrene-butadiene rubber, fluorine rubber, various copolymers thereof, and the like.

Typically, the binder may be included in an amount of 1 to 20 wt%, preferably 1 to 15 wt%, more preferably 1 wt% to 10 wt%, based on the total weight of solids excluding the solvent in the negative electrode mixture slurry.

The conductive material is a component for further improving the conductivity of the negative electrode active material, and may be added in an amount of 1 to 20 wt% based on the total weight of solids in the negative electrode mixture slurry. The conductive material is not particularly limited as long as it has conductivity without causing a chemical change in the battery, and for example, carbon powder such as carbon black, acetylene black, Ketjen black, channel black, furnace black, lamp black, or thermal black; graphite powder of natural graphite, artificial graphite, graphite, or the like, which has a very developed crystal structure; conductive fiber such as carbon fiber or metal fiber; conductive powder such as fluorocarbon powder, aluminum powder, and nickel powder; a conductive whisker such as zinc oxide and potassium titanate; a conductive metal oxide such as titanium oxide; a conductive material such as a polyphenylene derivative, and the like may be used.

The conductive material may be included in an amount of 1 to 20 wt%, preferably 1 to 15 wt%, more preferably 1 to 10 wt%, based on the total weight of solids excluding the solvent in the negative electrode mixture slurry.

The solvent may include water or an organic solvent such as N-methyl-2-pyrrolidone (NMP), and may be used in an amount such that a preferred viscosity is achieved when the negative electrode active material, and selectively, a binder and a conductive material, and the like are included. For example, the solvent may be included in an amount such that the concentration of solids including the negative electrode active material, and selectively, a binder and a conductive material, is 50 wt% to 95 wt%, preferably 50 wt% to 95 wt%, preferably 70 wt% to 90 wt%.

When a metal itself is used as the negative electrode, the negative electrode may be manufactured by physically bonding, roll-pressing, or depositing a metal on a metal thin film itself or the negative electrode current collector. The depositing method may be electrodeposition or chemical vapor deposition.

For example, the metal thin film itself, or the metal bonded/roll-pressed/deposited on the negative electrode current collector may be one metal selected from the group consisting of lithium (Li), nickel (Ni), tin (Sn), copper (Cu), and indium (In), or an alloy of two metals thereof.

### (3) Separator

In addition, as the separator, a common porous polymer film typically used as a separator, for example, a porous polymer film made of a polyolefin-based polymer, such as an ethylene homocopolymer, a propylene homocopolymer, an ethylene/butene copolymer, an ethylene/hexene copolymer, and an ethylene/methacrylate copolymer may be used alone, or a laminate thereof may be used. Alternatively, a common porous non-woven fabric, for example, a non-woven fabric made of glass fiber having a high melting point or polyethylene terephthalate fiber may be used, but the present invention is not limited thereto. Also, a coated separator including a ceramic component or a polymer material may be used to secure heat resistance or mechanical strength, and may be selectively used in a single-layered or a multi-layered structure.

The external shape of the lithium secondary battery of the present invention is not particularly limited, but may be a cylindrical shape using a can, a square shape, a pouch shape, a coin shape, or the like.

Hereinafter, the present invention will be described in more detail with reference to specific examples. However, the following examples are for illustrative purposes only to facilitate the understanding of the present invention, and do not limit the scope of the present invention.

### Examples

### Example 1

### (Preparation of non-aqueous electrolyte)

A non-aqueous solvent was prepared by dissolving LiPF₆ to 1.0 M, and vinylene carbonate (VC) to 0.5 wt% in an organic solvent (ethylene carbonate (EC) : ethylmethyl carbonate (EMC) = 3:7 volume ratio), and to the non-aqueous solvent, 0.1 g of a compound of Formula 1-2a below was introduced to prepare a non-aqueous electrolyte.

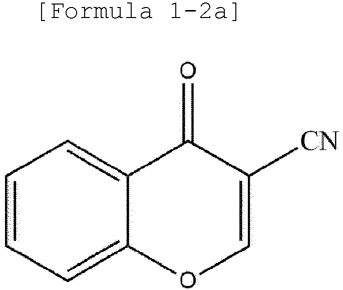

### (Manufacturing of lithium secondary battery)

A positive electrode active material (LiNi_{0.8}Co_{0.1}Mn_{0.1}Al₂O2): a conductive material (carbon nanotube) : a binder (polyvinylidene fluoride) were added at a weight ratio of 50:0.5:0.7 to N-methyl-2-pyrrolidone (NMP), which is a solvent, to prepare a positive electrode slurry (solid content of 75 wt%). The positive electrode slurry was applied on one surface of a positive electrode current collector (Al thin film) having a thickness of 15 µm, dried and then roll-pressed to manufacture a positive electrode.

A negative active material (graphite) : a conductive material (carbon nanotube) : a binder (mixed at a weight ratio of SBR/CMC = 3/1) were added to H₂O, which is a solvent, at a weight ratio of 20 : 0.1 : 1 to prepare a negative electrode slurry (solid content of 50 wt%). The negative electrode slurry was applied on one surface of a negative electrode current collector (Cu thin film) having a thickness of 8 µm, dried and then roll-pressed to manufacture a negative electrode.

A polyethylene separator was interposed between the positive electrode and the negative electrode manufactured in a dry room, and then the prepared non-aqueous electrolyte was injected thereto to manufacture a secondary battery.

### Example 2

A secondary battery was manufactured in the same manner as in Example 1 except that 1.0 g of a compound of Formula 1-2a above was introduced to 99.0 g of the non-aqueous solvent prepared in Example 1 to prepare a non-aqueous electrolyte.

### Example 3

A secondary battery was manufactured in the same manner as in Example 1 except that 2.0 g of a compound of Formula 1-2b below was introduced to 98.0 g of the non-aqueous solvent prepared in Example 1 to prepare a non-aqueous electrolyte.

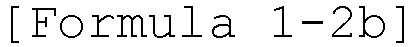

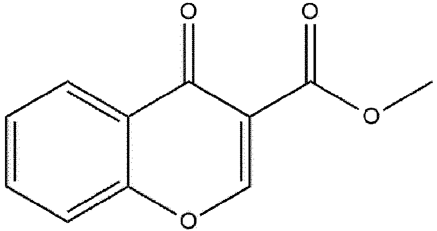

### Example 4

A secondary battery was manufactured in the same manner as in Example 1 except that, instead of a compound of Formula 1-2a, 0.5 g of a compound of Formula 1-2c below was introduced to 99.5 g of the non-aqueous solvent prepared in Example 1 to prepare a non-aqueous electrolyte.

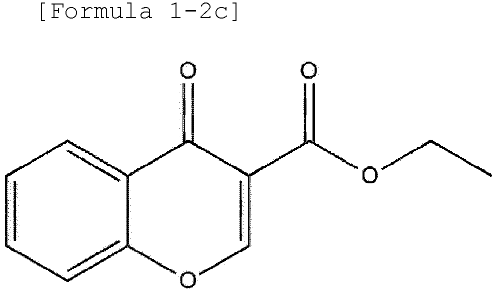

### Example 5

A secondary battery was manufactured in the same manner as in Example 1 except that, instead of a compound of Formula 1-2a, 0.5 g of a compound of Formula 1-2d below was introduced to 99.5 g of the non-aqueous solvent prepared in Example 1 to prepare a non-aqueous electrolyte.

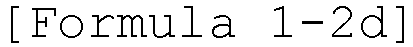

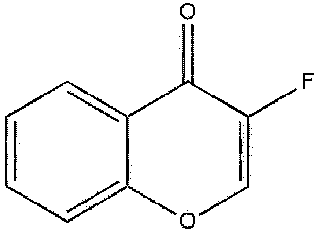

### Example 6

A secondary battery was manufactured in the same manner as in Example 1 except that, instead of a compound of Formula 1-2a, 0.5 g of a compound of Formula 1-2h below was introduced to 99.5 g of the non-aqueous solvent prepared in Example 1 to prepare a non-aqueous electrolyte.

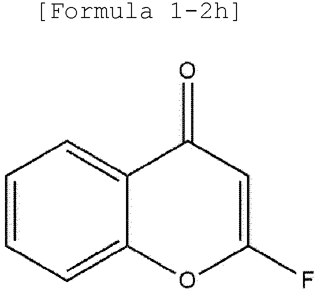

### Comparative Example 1

A secondary battery was manufactured in the same manner as in Example 1 except that 100 g of the non-aqueous solvent prepared in Example 1 was used to prepare a non-aqueous electrolyte.

### Comparative Example 2

A secondary battery was manufactured in the same manner as in Example 1 except that, instead of a compound of Formula 1-2a, 0.5 g of a compound of Formula A below was introduced to 99.5 g of the non-aqueous solvent prepared in Example 1 to prepare a non-aqueous electrolyte.

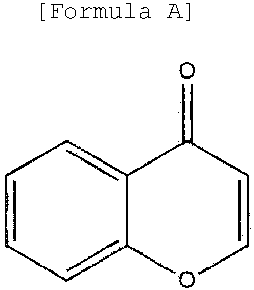

### Comparative Example 3

A secondary battery was manufactured in the same manner as in Example 1 except that, instead of a compound of Formula 1-2a, 0.5 g of a compound of Formula B below was introduced to 99.5 g of the non-aqueous solvent prepared in Example 1 to prepare a non-aqueous electrolyte.

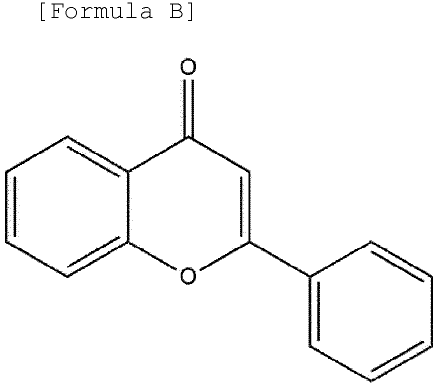

### Experimental Example 1 - Evaluation of high-temperature cycle properties

For each of the secondary battery batteries manufactured in Examples 1 to 6 and Comparative Examples 1 to 3, cycle properties were evaluated.

Specifically, each of the batteries manufactured in Examples 1 to 6 and Comparative Examples 1 to 3 was charged to 4.2 V with a constant current of 0.33 C at 45°C, and then charged to 1/40 C with a constant voltage, and discharged to 2.5 V with a constant current of 0.33 C, which was set to one cycle, and then 100 cycles of the charging/discharging were performed to measure a resistance increase rate after 100 cycles based on an initial resistance was measured. The results are shown in Table 1 below.

**[Table 1]**

| | Resistance increase rate (%) |
|---|---|
| Example 1 | 2.5 |
| Example 2 | 5.8 |
| Example 3 | 5.6 |
| Example 4 | 3.2 |
| Example 5 | 2.9 |
| Example 6 | 3.1 |
| Comparative Example 1 | 9.3 |
| Comparative Example 2 | 8.2 |
| Comparative Example 3 | 7.9 |

As shown in Table 1, Examples 1 to 6 in which the additive for a non-aqueous electrolyte of the present invention was used had a lower resistance increase rate than that of Comparative Example 1 in which the additive was not used, and thus, had excellent lifespan properties. In addition, Examples 1 to 6 using the additive for a non-aqueous electrolyte of the present invention, the additive in which an electron withdrawing group is substituted at positions 2 and 3 of chromone, had excellent lifespan properties since the resistance increase rate thereof was lower than that of Comparative Example 2 using the additive of Formula A in which there is no substituent at positions 2 and 3 of chromone and that of Comparative Example 3 using the additive of Formula B in which an electron donating group was substituted at positions 2 and 3 of chromone.

### Experimental Example 2 - Evaluation of high-temperature storage properties

For each of the secondary battery batteries manufactured in Examples 1 to 6 and Comparative Examples 1 to 3, high-temperature storage properties were evaluated.

Specifically, the secondary batteries of Examples 1 to 6 and Comparative Examples 1 to 3 were each fully charged to 4.2 V, and then stored at 60°C for 4 weeks.

Before the storage, the volume of each of the fullycharged secondary batteries was measured and then set to an initial thickness of the secondary battery.

After 4 weeks, the volume of each of the stored secondary battery was measured to calculate a thickness increased during the 4-week storage period. The percentage ratio of the increased volume to the initial volume of the secondary battery was calculated to derive a thickness increase rate after the 4 weeks. The results are shown in Table 2 below.

**[Table 2]**

| | Thickness increase rate (%) |
|---|---|
| Example 1 | 2.6 |
| Example 2 | 4.8 |
| Example 3 | 7.7 |
| Example 4 | 4.5 |
| Example 5 | 3.8 |
| Example 6 | 4.1 |
| Comparative Example 1 | 10.2 |
| Comparative Example 2 | 9.9 |
| Comparative Example 3 | 8.7 |

As shown in Table 2 above, the secondary batteries of Examples 1 to 6 had a lower thickness increase rate after the 4 weeks than that of the secondary battery of Comparative Example 1, and thus, had less gas generation at high temperatures. In addition, Examples 1 to 6 using the additive for a non-aqueous electrolyte of the present invention, the additive in which an electron withdrawing group is substituted at positions 2 and 3 of chromone, had less gas generation since the volume increase rate thereof was lower than that of Comparative Example 2 using the additive of Formula A in which there is no substituent at positions 2 and 3 of chromone and that of Comparative Example 3 using the additive of Formula B in which an electron donating group was substituted at positions 2 and 3 of chromone.

## Claims

1. A non-aqueous electrolyte comprising an additive for a non-aqueous electrolyte represented by Formula 1 below: 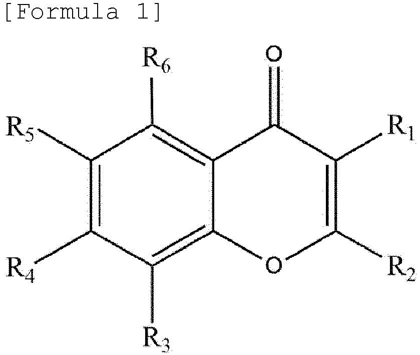
wherein in Formula 1 above, R₁ and R₂ are each independently any one selected from the group consisting of H; F; a nitrile group; a thiocyanate group; an isothiocyanate group; an isocyanate group; an alkoxycarbonyl group having 1 to 10 carbon atoms; an alkylcarbonyl group having 1 to 10 carbon atoms; a sulfonyl group substituted with an alkyl group having 1 to 10 carbon atoms, an alkenyl group having 2 to 10 carbon atoms, or an alkynyl group having 2 to 10 carbon atoms; an oxysulfonyl group substituted with an alkyl group having 1 to 10 carbon atoms, an alkenyl group having 2 to 10 carbon atoms, or an alkynyl group having 2 to 10 carbon atoms; CF₃; OCF₃; COOCF₃; and SO₂CF₃, wherein R₁ and R₂ are not H at the same time, and
R₃ to R₆ are each independently any one selected from the group consisting of H, an alkyl group having 1 to 10 carbon atoms, an alkenyl group having 2 to 10 carbon atoms, an alkynyl group having 2 to 10 carbon atoms, an alkoxy group having 1 to 10 carbon atoms, a cycloalkyl group having 3 to 12 carbon atoms, a cycloalkenyl group having 3 to 12 carbon atoms, and an aryl group having 6 to 20 carbon atoms,
wherein the alkoxycarbonyl is a -COOR, in which R is an alkyl group having 1 to 10 carbon atoms, and,
wherein the non-aqueous electrolyte further comprises a lithium salt.

2. The non-aqueous electrolyte of claim 1, wherein the compound of Formula 1 above is an additive for a non-aqueous electrolyte represented by Formula 1-1 below. 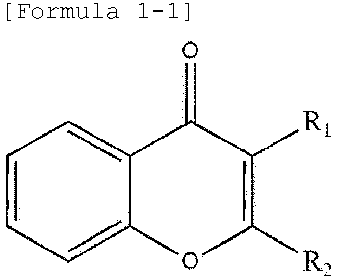 wherein in Formula 1-1 above, R₁ and R₂ are each independently any one selected from the group consisting of H, F, an alkoxycarbonyl group having 1 to 10 carbon atoms, and a nitrile group, wherein R₁ and R₂ are not H at the same time,
wherein the alkoxycarbonyl is a -COOR, in which R is an alkyl group having 1 to 10 carbon atoms.

3. The non-aqueous electrolyte of claim 1, wherein the compound of Formula 1 above is any one selected from the group consisting of additives for non-aqueous electrolytes represented by Formulas 1-2a to 1-2h.

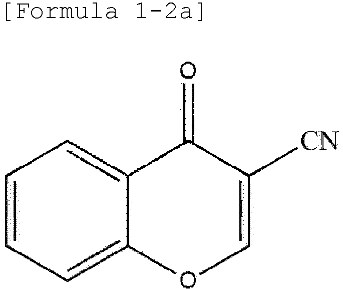

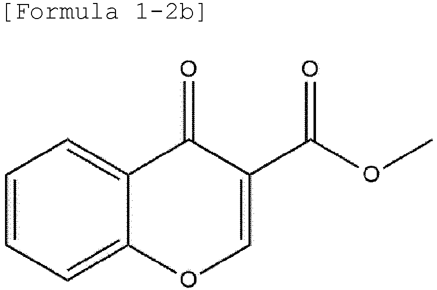

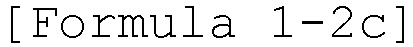

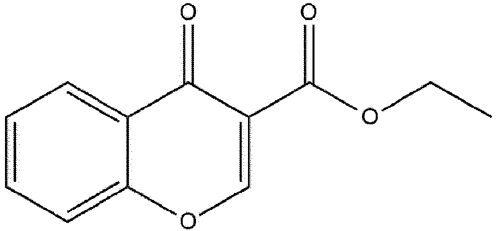

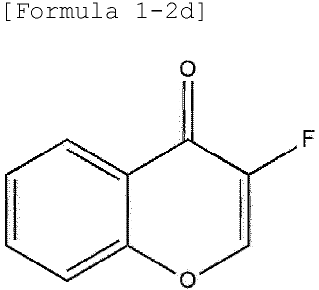

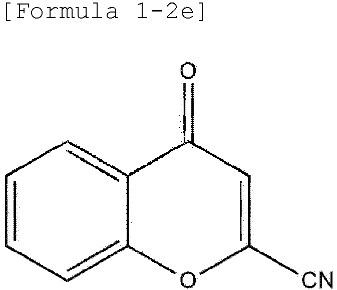

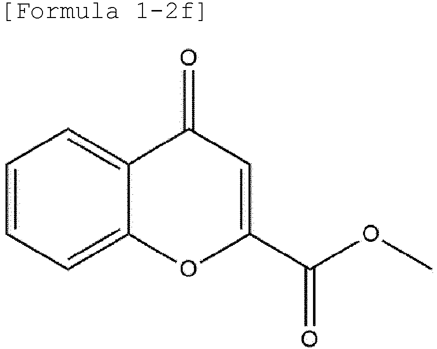

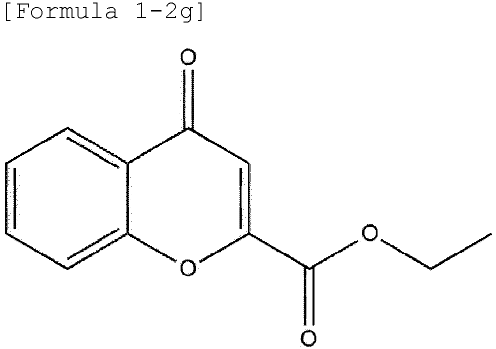

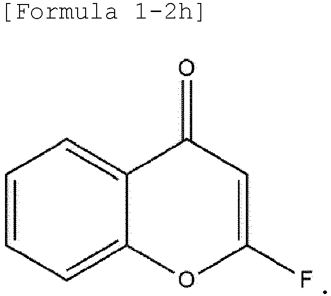

4. The non-aqueous electrolyte of claim 1, wherein the additive for a non-aqueous electrolyte is included in a content of 0.01 parts by weight to 10 parts by weight based on 100 parts by weight of the non-aqueous electrolyte.

5. The non-aqueous electrolyte of claim 1, wherein the lithium salt is included at a concentration of 0.5 M to 2.0 M.

6. The non-aqueous electrolyte of claim 1, wherein the lithium salt is one or more selected from the group consisting of LiCl, LiBr, LiI, LiBF₄, LiClO₄, LiB₁₀Cl₁₀, LiAlCl₄, LiAlO₂, LiPF₆, LiCF₃SO₃, LiCH₃CO₂, LiCF₃CO₂, LiAsF₆, LiSbF₆, LiCH₃SO₃, LiN(SO₂F)₂, LiN(SO₂CF₂CF₃)₂, and LiN(SO₂CF₃)₂.

7. The non-aqueous electrolyte of claim 1, further comprising an organic solvent.

8. The non-aqueous electrolyte of claim 7, wherein the organic solvent comprises at least one organic solvent selected from the group consisting of a cyclic carbonate-based organic solvent, a linear carbonate-based organic solvent, a linear ester-based organic solvent, and a cyclic ester-based organic solvent.

9. The non-aqueous electrolyte of claim 1, further comprising, as an additive, one or more compounds selected from the group consisting of a cyclic carbonate-based compound, a halogen-substituted carbonate-based compound, a sultone-based compound, a sulfate-based compound, a phosphate-based compound, a borate-based compound, a nitrile-based compound, a benzene-based compound, an amine-based compound, a silane-based compound, and a lithium salt-based compound.

10. A lithium secondary battery comprising the non-aqueous electrolyte of any one of claims 1 to 9.

## Patentansprüche

1. Nichtwässriger Elektrolyt, umfassend ein durch die nachstehende Formel 1 dargestelltes Additiv für einen nichtwässrigen Elektrolyten: 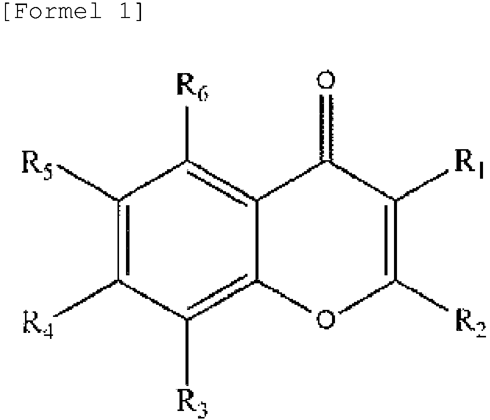
wobei in der obigen Formel 1 R₁ und R₂ jeweils unabhängig eines, ausgewählt aus der Gruppe, bestehend aus H, F, einer Nitrilgruppe, einer Thiocyanatgruppe, einer Isothiocyanatgruppe, einer Isocyanatgruppe, einer Alkoxycarbonylgruppe mit 1 bis 10 Kohlenstoffatomen, einer Alkylcarbonylgruppe mit 1 bis 10 Kohlenstoffatomen, einer Sulfonylgruppe, die mit einer Alkylgruppe mit 1 bis 10 Kohlenstoffatomen, einer Alkenylgruppe mit 2 bis 10 Kohlenstoffatomen oder einer Alkinylgruppe mit 2 bis 10 Kohlenstoffatomen substituiert ist, einer Oxysulfonylgruppe, die mit einer Alkylgruppe mit 1 bis 10 Kohlenstoffatomen, einer Alkenylgruppe mit 2 bis 10 Kohlenstoffatomen oder einer Alkinylgruppe mit 2 bis 10 Kohlenstoffatomen substituiert ist, CF₃, OCF₃, COOCF₃ und SO₂CF₃, sind, wobei R₁ und R₂ nicht gleichzeitig H sind, und
R₃ bis R₆ jeweils unabhängig eines, ausgewählt aus der Gruppe, bestehend aus H, einer Alkylgruppe mit 1 bis 10 Kohlenstoffatomen, einer Alkenylgruppe mit 2 bis 10 Kohlenstoffatomen, einer Alkinylgruppe mit 2 bis 10 Kohlenstoffatomen, einer Alkoxygruppe mit 1 bis 10 Kohlenstoffatomen, einer Cycloalkylgruppe mit 3 bis 12 Kohlenstoffatomen, einer Cycloalkenylgruppe mit 3 bis 12 Kohlenstoffatomen und einer Arylgruppe mit 6 bis 20 Kohlenstoffatomen, sind,
wobei die Alkoxycarbonylgruppe ein -COOR ist, worin R eine Alkylgruppe mit 1 bis 10 Kohlenstoffatomen ist, und
wobei der nichtwässrige Elektrolyt ferner ein Lithiumsalz umfasst.

2. Nichtwässriger Elektrolyt nach Anspruch 1, wobei die Verbindung der obigen Formel 1 ein durch die nachstehende Formel 1-1 dargestelltes Additiv für einen nichtwässrigen Elektrolyten ist: 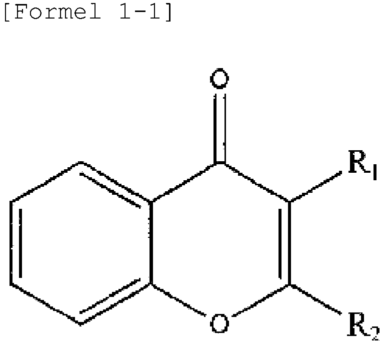
wobei in der obigen Formel 1-1 R₁ und R₂ jeweils unabhängig eines, ausgewählt aus der Gruppe, bestehend aus H, F, einer Alkoxycarbonylgruppe mit 1 bis 10 Kohlenstoffatomen und einer Nitrilgruppe, sind, wobei R₁ und R₂ nicht gleichzeitig H sind,
wobei die Alkoxycarbonylgruppe ein -COOR ist, worin R eine Alkylgruppe mit 1 bis 10 Kohlenstoffatomen ist.

3. Nichtwässriger Elektrolyt nach Anspruch 1, wobei die Verbindung der obigen Formel 1 eine, ausgewählt aus der Gruppe, bestehend aus durch die Formeln 1-2a bis 1-2h dargestellten Additiven für nichtwässrige Elektrolyte, ist:

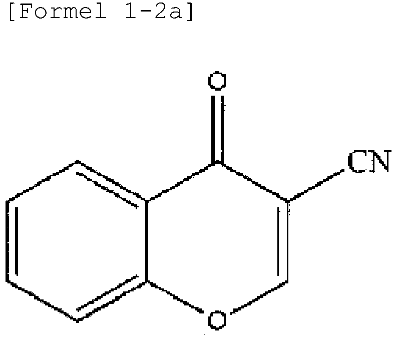

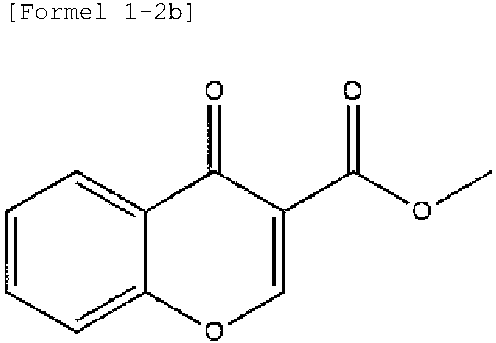

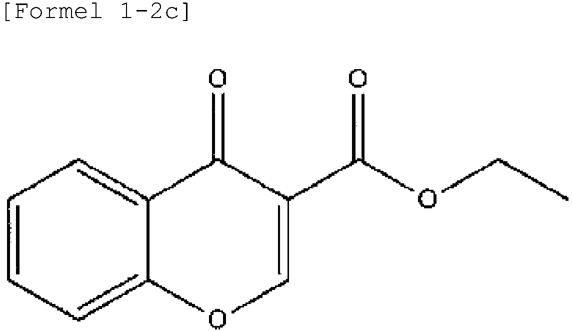

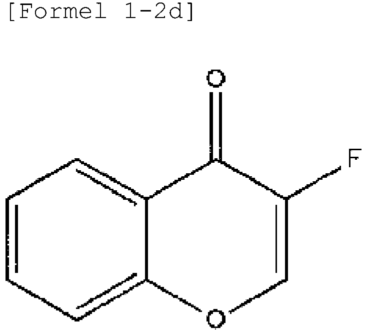

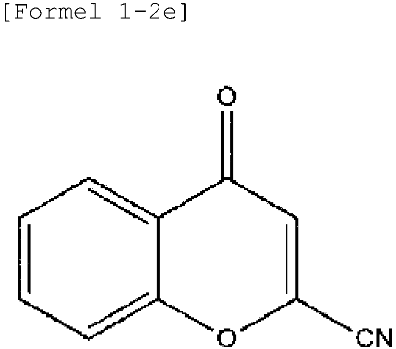

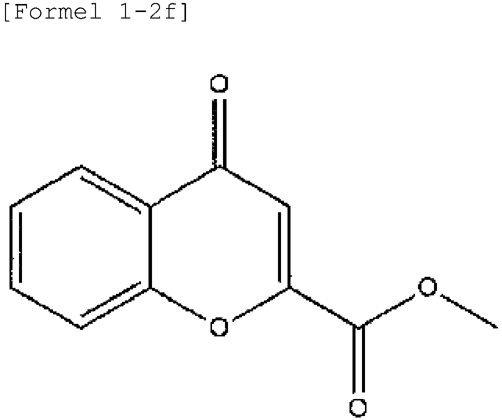

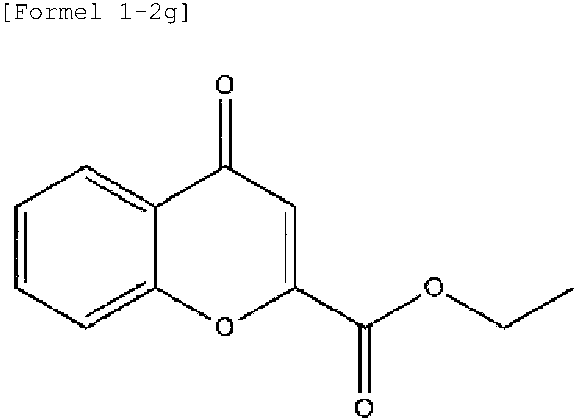

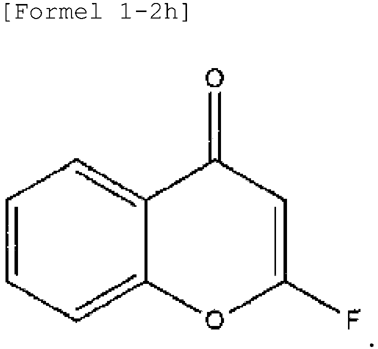

4. Nichtwässriger Elektrolyt nach Anspruch 1, wobei das Additiv für einen nichtwässrigen Elektrolyten in einem Gehalt von 0,01 Gewichtsteilen bis 10 Gewichtsteilen, bezogen auf 100 Gewichtsteile des nichtwässrigen Elektrolyten, enthalten ist.

5. Nichtwässriger Elektrolyt nach Anspruch 1, wobei das Lithiumsalz bei einer Konzentration von 0,5 M bis 2,0 M enthalten ist.

6. Nichtwässriger Elektrolyt nach Anspruch 1, wobei das Lithiumsalz eines oder mehrere, ausgewählt aus der Gruppe, bestehend aus LiCl, LiBr, LiI, LiBF₄, LiClO₄, LiB₁₀Cl₁₀, LiAlCl₄, LiAlO₂, LiPF₆, LiCF₃SO₃, LiCH₃CO₂, LiCF₃CO₂, LiAsF₆, LiSbF₆, LiCH₃SO₃, LiN(SO₂F)₂, LiN(SO₂CF₂CF₃)₂ und LiN(SO₂CF₃)₂, ist.

7. Nichtwässriger Elektrolyt nach Anspruch 1, ferner umfassend ein organisches Lösungsmittel.

8. Nichtwässriger Elektrolyt nach Anspruch 7, wobei das organische Lösungsmittel mindestens ein organisches Lösungsmittel, ausgewählt aus der Gruppe, bestehend aus einem organischen Lösungsmittel auf Basis eines cyclischen Carbonats, einem organischen Lösungsmittel auf Basis eines linearen Carbonats, einem organischen Lösungsmittel auf Basis eines linearen Esters und einem organischen Lösungsmittel auf Basis eines cyclischen Esters, umfasst.

9. Nichtwässriger Elektrolyt nach Anspruch 1, ferner umfassend als ein Additiv eine oder mehrere Verbindungen, ausgewählt aus der Gruppe, bestehend aus einer Verbindung auf Basis eines cyclischen Carbonats, einer Verbindung auf Basis eines halogensubstituierten Carbonats, einer Verbindung auf Sultonbasis, einer Verbindung auf Sulfatbasis, einer Verbindung auf Phosphatbasis, einer Verbindung auf Boratbasis, einer Verbindung auf Nitrilbasis, einer Verbindung auf Benzolbasis, einer Verbindung auf Aminbasis, einer Verbindung auf Silanbasis und einer Verbindung auf Basis eines Lithiumsalzes.

10. Lithiumsekundärbatterie, umfassend den nichtwässrigen Elektrolyten gemäß einem der Ansprüche 1 bis 9.

## Revendications

1. Électrolyte non aqueux comprenant un additif pour un électrolyte non aqueux représenté par la Formule 1 ci-dessous : 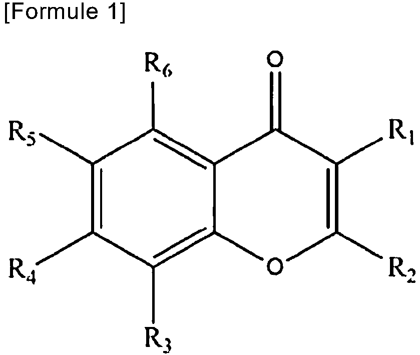
dans lequel, dans la Formule 1 ci-dessus, R₁ et R₂ sont chacun indépendamment l'un quelconque choisis dans le groupe consistant en H ; F ; un groupe nitrile ; un groupe thiocyanate ; un groupe isothiocyanate ; un groupe isocyanate ; un groupe alcoxycarbonyle présentant 1 à 10 atomes de carbone ; un groupe alkylcarbonyle présentant 1 à 10 atomes de carbone ; un groupe sulfonyle substitué par un groupe alkyle présentant 1 à 10 atomes de carbone, un groupe alcényle présentant 2 à 10 atomes de carbone ou un groupe alcynyle présentant 2 à 10 atomes de carbone ; un groupe oxysulfonyle substitué par un groupe alkyle présentant 1 à 10 atomes de carbone, un groupe alcényle présentant 2 à 10 atomes de carbone ou un groupe alcynyle présentant 2 à 10 atomes de carbone ; CF₃ ; OCF₃ ;COOCF₃ ; et SO₂CF₃, dans lequel R₁ et R₂ ne sont pas H en même temps, et
R₃ à R₆ sont chacun indépendamment l'un quelconque choisis dans le groupe consistant en H, un groupe alkyle présentant 1 à 10 atomes de carbone, un groupe alcényle présentant 2 à 10 atomes de carbone, un groupe alcynyle présentant 2 à 10 atomes de carbone, un groupe alcoxy présentant 1 à 10 atomes de carbone, un groupe cycloalkyle présentant 3 à 12 atomes de carbone, un groupe cycloalcényle présentant 3 à 12 atomes de carbone et un groupe aryle présentant 6 à 20 atomes de carbone,
dans lequel l'alcoxycarbonyle est un -COOR, dans lequel R est un groupe alkyle présentant 1 à 10 atomes de carbone, et,
dans lequel l'électrolyte non aqueux comprend en outre un sel de lithium.

2. Électrolyte non aqueux selon la revendication 1, dans lequel le composé de Formule 1 ci-dessus est un additif pour un électrolyte non aqueux représenté par la Formule 1-1 ci-dessous. 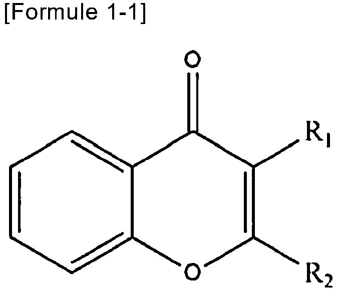
dans lequel, dans la Formule 1-1 ci-dessus, R₁ et R₂ sont chacun indépendamment l'un quelconque choisis dans le groupe consistant en H, F, un groupe alcoxycarbonyle présentant 1 à 10 atomes de carbone, et un groupe nitrile, dans lequel R₁ et R₂ ne sont pas H en même temps,
dans lequel l'alcoxycarbonyle est un -COOR, dans lequel R est un groupe alkyle présentant 1 à 10 atomes de carbone.

3. Électrolyte non aqueux selon la revendication 1, dans lequel le composé de Formule 1 ci-dessus est l'un quelconque choisi dans le groupe consistant en des additifs pour électrolytes non aqueux représentés par les Formules 1-2a à 1-2h.

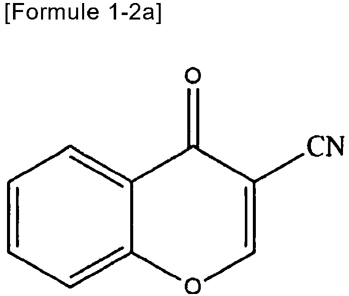

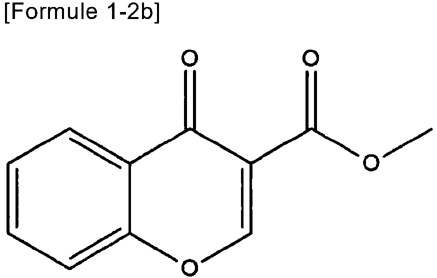

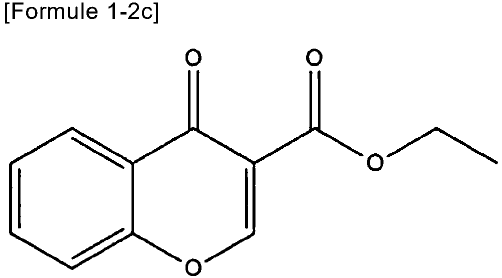

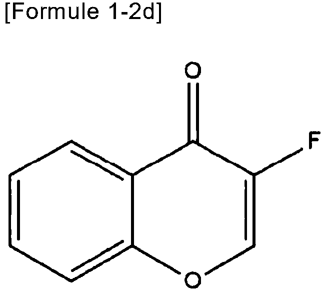

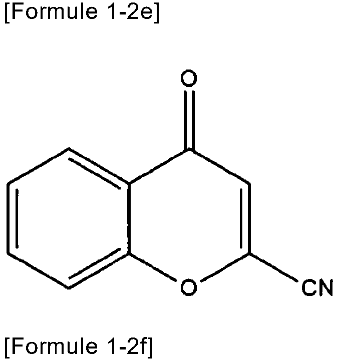

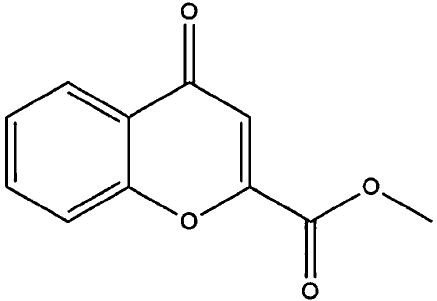

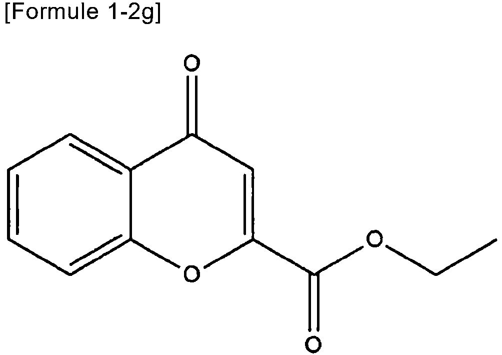

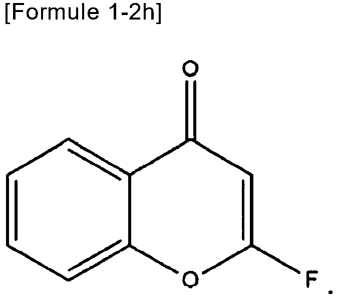

4. Électrolyte non aqueux selon la revendication 1, dans lequel l'additif pour un électrolyte non aqueux est inclus dans une teneur comprise entre 0,01 partie en poids et 10 parties en poids sur la base de 100 parties en poids de l'électrolyte non aqueux.

5. Électrolyte non aqueux selon la revendication 1, dans lequel le sel de lithium est inclus à une concentration comprise entre 0,5 M et 2,0 M.

6. Électrolyte non aqueux selon la revendication 1, dans lequel le sel de lithium est un ou plusieurs éléments choisis dans le groupe consistant en LiCl, LiBr, Lil, LiBF₄, LiClO₄, LiB₁₀Cl₁₀, LiAlCl₄, LiAlO₂, LiPF₆, LiCF₃SO₃, LiCH₃CO₂, LiCF₃CO₂, LiAsF₆, LiSbF₆, LiCH₃SO₃, LiN(SO₂F)₂, LiN(SO₂CF₂CF₃)₂, et LiN(SO₂CF₃)₂.

7. Électrolyte non aqueux selon la revendication 1, comprenant en outre un solvant organique.

8. Électrolyte non aqueux selon la revendication 7, dans lequel le solvant organique comprend au moins un solvant organique choisi dans le groupe consistant en un solvant organique à base de carbonate cyclique, un solvant organique à base de carbonate linéaire, un solvant organique à base d'ester linéaire et un solvant organique à base d'ester cyclique.

9. Électrolyte non aqueux selon la revendication 1, comprenant en outre, en tant qu'additif, un ou plusieurs composés choisis dans le groupe consistant en un composé à base de carbonate cyclique, un composé à base de carbonate substitué par un halogène, un composé à base de sultone, un composé à base de sulfate, un composé à base de phosphate, un composé à base de borate, un composé à base de nitrile, un composé à base de benzène, un composé à base d'amine, un composé à base de silane, et un composé à base de sel de lithium.

10. Batterie secondaire au lithium comprenant l'électrolyte non aqueux selon l'une quelconque des revendications 1 à 9.
